(19) Europäisches Patentamt
European Patent Office
Office européen des brevets

(11) EP 4 257 124 A1

(12) **EUROPEAN PATENT APPLICATION**
published in accordance with Art. 153(4) EPC

(43) Date of publication:
**11.10.2023 Bulletin 2023/41**

(21) Application number: **21900958.6**

(22) Date of filing: **30.11.2021**

(51) International Patent Classification (IPC):
***A61K 9/20*** *(2006.01)* ***A61K 31/4155*** *(2006.01)*

(52) Cooperative Patent Classification (CPC):
**A61K 9/20; A61K 31/4155**

(86) International application number:
**PCT/KR2021/017843**

(87) International publication number:
**WO 2022/119270 (09.06.2022 Gazette 2022/23)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **01.12.2020 KR 20200165790**

(71) Applicant: **Lg Chem, Ltd.**
**Seoul 07336 (KR)**

(72) Inventors:
- **YOO, Seok Cheol**
  **Daejeon 34122 (KR)**
- **JANG, Joomyung**
  **Daejeon 34122 (KR)**
- **KIM, Ree Sun**
  **Daejeon 34122 (KR)**
- **SEO, Jin A**
  **Daejeon 34122 (KR)**

(74) Representative: **Hoffmann Eitle**
**Patent- und Rechtsanwälte PartmbB**
**Arabellastraße 30**
**81925 München (DE)**

(54) **ORAL FORMULATION COMPRISING 1-(3-CYANO-1-ISOPROPYL-INDOLE-5-YL)PYRAZOLE-4-CARBOXYLIC ACID AND METHOD FOR PREPARING SAME**

(57) The present invention relates to an oral formulation comprising an API selected from 1-(3-cyano-1-isopropyl-indol-5-yl)pyrazol-4-carboxylic acid or a pharmaceutically acceptable salt thereof in high content.

Since the oral formulation according to the present invention has a high content and excellent physical properties by comprising a glidant in excipients, economic efficiency and the convenience of administration can be increased.

【Figure 1】

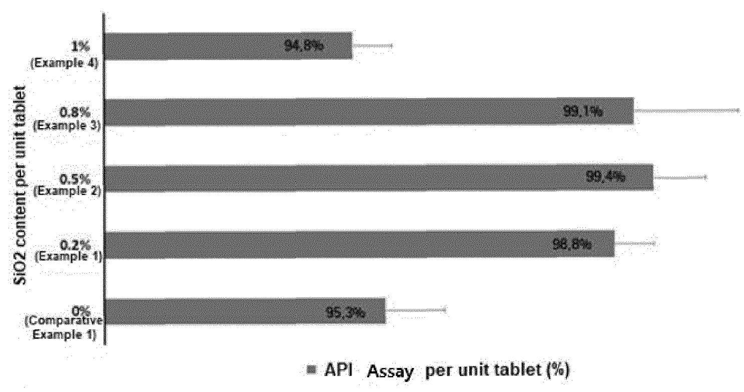


EP 4 257 124 A1

## Description

[Technical Field]

The present application claims the benefit of priority based on Korean Patent Application No. 10-2020-0165790 filed on December 1, 2020, the entire disclosure of which are incorporated herein by reference its entirely.

The present invention relates to an oral formulation with excellent physical properties, which comprising an active pharmaceutical ingredient (API) selected from 1-(3-cyano-1-isopropyl-indol-5-yl)pyrazol-4-carboxylic acid or a pharmaceutically acceptable salt thereof with high content, which is useful as a xanthine oxidase inhibitor that can prevent the deposition of uric acid in the body, and to a preparation method thereof.

[Background Art]

Xanthine oxidase is an enzyme that converts hypoxanthine to xanthine, and also converts the formed xanthine to uric acid. It is known that when there is too much uric acid in the body, it causes various diseases, including gout and the like.

Therefore, substances that inhibit the activity of xanthine oxidase can effectively treat xanthine oxidase-related diseases such as hyperuricacidemia, gout, heart failure, cardiovascular diseases, high blood pressure, diabetes, kidney diseases, joint diseases, and inflammatory bowel disease.

Meanwhile, with respect to a substance that inhibits the activity of xanthine oxidase, KR 10-1751325 (Patent Document 1) provides 1-(3-cyano-1-isopropyl-indol-5-yl)pyrazol-4-carboxylic acid, and a method for preparing the compound, and KR 10-1424013 (Patent Document 2) provides various types of crystalline forms obtained by using various solvents, and a preparation method thereof.

[Formula 1]

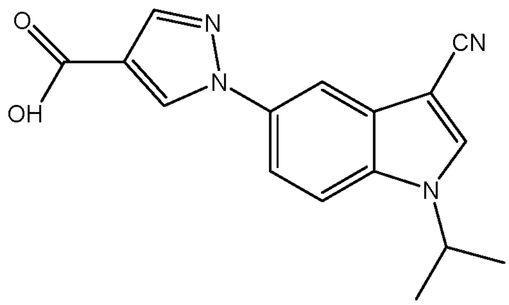

However, it has not been reported for an oral formulation comprising 1-(3-cyano-1-isopropyl-indol-5-yl)pyrazol-4-carboxylic acid or a pharmaceutically acceptable salt thereof as an active pharmaceutical ingredient (API), and in particular, there has been no report on the oral formulation comprising the API with high content.

Formulations comprising API with high content cannot generally be said to have excellent fluidity and tableting properties. Accordingly, in order to overcome these disadvantages, it is common to use wet granulation or dry granulation method or to increase the amounts of excipients to reduce the API ratio.

However, there is a problem that the high content formulation can increase the manufacturing time and production cost by performing a granulation process for improving fluidity, as well as increase the total weight and volume of the formulation by increasing the amount of excipients. So conventional high content formulation shows increasing the production cost and lowering the patient’s convenience in taking. In addition, it is common knowledge in the art that even if a granulation process is added or the amounts of excipients is increased to prepare the high content formulation, the formulation assay may be affected by the imbalance between the excipients and the high content API.

Therefore, it is necessary to develop an oral formulation with excellent physical properties while comprising 1-(3-cyano-1-isopropyl-indol-5-yl)pyrazol-4-carboxylic acid or a pharmaceutically acceptable salt thereof as API with high content.

[Prior Art Documents]

[Patent Documents]

1. KR 10-1751325 (June 21, 2017), Novel compounds effective as xanthine oxidase inhibitors, method for preparing the same, and pharmaceutical composition comprising the same
2. KR 10-1424013 (July 22, 2014), 1-(3-cyano-1-isopropyl-indol-5-yl)pyrazole-4-carboxyl ic acid crystalline form and the producing method thereof

[Disclosure]

[Technical Problem]

It is an object of the present invention to provide an oral formulation having excellent physical properties, comprising an excess of 1-(3-cyano-1-isopropyl-indol-5-yl)pyrazol-4-carboxylic acid or a pharmaceutically acceptable salt thereof, as an API.

[Technical Solution]

The present invention provides an oral formulation with excellent physical properties, comprising an API selected from 1-(3-cyano-1-isopropyl-indol-5-yl)pyrazol-4-carboxylic acid or a pharmaceutically acceptable salt thereof, and excipients, in particular a glidant in the excipients, and a method for preparing the oral formulation.
The glidant comprised in the oral formulation of the present invention is $SiO_2$ selected from the group consisting of colloidal silicon dioxide, hydrated silicon dioxide and combinations thereof, and the content of the glidant per formulation is 0.2 to 1% by weight, or 0.2 to 0.8% by weight, based on the total weight of the formulation.
The content of API comprised in the oral formulation of the present invention is 30 to 50% by weight based on the total weight of the formulation.
The API assay of the oral formulation of the present invention is 95% or more.
The oral formulation of the present invention is used for the treatment or prophylaxis of xanthine oxidase-related diseases selected from the group consisting of hyperuricacidemia, gout, heart failure, cardiovascular diseases, high blood pressure, diabetes, kidney diseases, inflammation, joint diseases, and inflammatory bowel disease.

[Advantageous Effects]

The oral formulation comprising 1-(3-cyano-1-isopropyl-indol-5-yl)pyrazol-4-carboxylic acid or a pharmaceutically acceptable salt thereof as an API according to the present invention has excellent physical properties and maintain high formulation assay with a glidant in the excipients despite the high API content.
In addition, the oral formulation according to the present invention comprises a high content of 1-(3-cyano-1-isopropyl-indol-5-yl)pyrazol-4-carboxylic acid or a pharmaceutically acceptable salt thereof, which is an API, and thus is not only economical, but also can increase the convenience of administration.

[Description of Drawings]

FIG. 1 shows API assay per unit tablet depending on glidant ($SiO_2$) content.

[Best Mode]

Hereinafter, the present invention will be described in more detail.
All technical terms used in the present invention, unless otherwise defined, are used in the same meaning as commonly understood by those of ordinary skill in the related field of the present invention. In addition, although preferred methods and samples are described herein, similar or equivalent ones are also included in the scope of the present invention. The disclosure of all publications incorporated herein by reference are hereby incorporated by reference in their entirety.
The inventors of the present invention have continued research in various ways in order to increase the content of the API and maintain or increase the physical properties in the oral formulation comprising 1-(3-cyano-1-isopropyl-indol-5-yl)pyrazol-4-carboxylic acid or a pharmaceutically acceptable salt thereof as API. And as a result, the inventors of the present invention have developed an oral formulation with high API assay while excellent physical properties comprising a glidant in excipients.
In this case, even if it comprises an excess of API compared to the oral formulation that does not comprise the glidant, since the physical properties are maintained when formulated as an uncoated tablet, there is no need to increase the content of excipients according to the increase of API content, and thus it has the advantage of being economical as well as being able to make a high-content oral formulation with increased convenience of administration without

increasing the size of the formulation. In addition, there is an advantage that since it has a high API assay, the quality of the final product is also excellent.

Therefore, the present invention provides an oral formulation comprising a high content of the API selected from 1-(3-cyano-1-isopropyl-indol-5-yl)pyrazol-4-carboxylic acid or a pharmaceutically acceptable salt thereof, with a glidant in excipients.

As used herein, "pharmaceutically acceptable salt" refers to a salt form of a compound that does not cause serious irritation to the organism to which the compound is administered and does not impair the biological activities and physical properties of the compound. 1-(3-cyano-1-isopropyl-indol-5-yl)pyrazol-4-carboxylic acid, which is the API comprised in the oral formulation of the present invention, can be converted to its salt by conventional methods.

As used herein, the term "API assay" or "assay" refers to the amount of an active ingredient in a formulation or tablet. The assay is an important quality characteristic of the final product.

In oral formulations such as tablets or granular capsules, it is common to minimize the size per unit dosage form for easy swallowing even if a high content API is included. However, it is the technical common knowledge of those of ordinary skill in the art that when the API is comprised in high content, not only physical properties such as fluidity and tableting properties are generally impaired, but also the assay of the formulation may be affected by the imbalance between the high content API and each excipient.

In fact, in an oral formulation comprising 1-(3-cyano-1-isopropyl-indol-5-yl)pyrazol-4-carboxylic acid as an API, the acceptable content of the API in the oral formulation (especially tablets) with pharmaceutically acceptable physical properties is only around 30%, and when more than 30% of the content is included, there was a problem that physical properties such as tableting properties are rapidly deteriorated.

In the present invention, various studies were conducted by combining various excipients for oral formulations with excellent or good physical stability while comprising an API with high content, selected from 1-(3-cyano-1-isopropyl-indol-5-yl)pyrazol-4-carboxylic acid or a pharmaceutically acceptable salt there.

As a result, in the case of including a glidant as an excipient, an oral formulation with high assay as well as excellent physical stability was developed despite including a high content of API.

Accordingly, one aspect of the present invention provides an oral formulation comprising i) 1-(3-cyano-1-isopropyl-indol-5-yl)pyrazol-4-carboxylic acid or a pharmaceutically acceptable salt thereof as an API, and ii) a glidant in excipients.

In the present invention, the glidant may be comprised in an amount of greater than 0 % by weight and less than 1 % by weight, 0.1 % by weight or more and less than 1 % by weight, 0.2 % by weight or more and less than 1 % by weight, greater than 0 % by weight and 0.9 % by weight or less, 0.1 % by weight or more and 0.9 % by weight or less, 0.2 % by weight or more and 0.9 % by weight or less, greater than 0 % by weight and 0.8 % by weight or less, 0.1 % by weight or more and 0.8 % by weight or less, or 0.2 % by weight or more and 0.8 % by weight or less, by weight based on the total weight of the formulation. In addition, the glidant may be comprised in an amount of 0.1 % by weight, 0.2 % by weight, 0.3 % by weight, 0.4 % by weight, 0.5 % by weight, 0.6 % by weight, 0.7 % by weight, 0.8 % by weight, or 0.9 % by weight based on the total weight of the formulation.

In the present invention, the glidant may be selected from the group consisting of colloidal silicon dioxide, hydrated silicon dioxide, talc and combinations thereof, but is not limited thereto. However, when talc is used as a glidant, since hardness tends to decrease and friability tends to increase when formulated as tablets, it is preferable to use a $SiO_2$ series such as colloidal silicon dioxide, hydrated silicon dioxide, or a combination thereof as the glidant.

In the present invention, the oral formulation further comprises one or more excipients selected from a pharmaceutically acceptable diluent, disintegrant, binder, stabilizer, lubricant and the like as an excipient.

The diluent, disintegrant, binder, stabilizer, lubricant and the like may be any ones known to be commonly used in the art. The diluent may be used in an amount of 30 to 50 % by weight, 40 to 50 % by weight, or 45 to 50 % by weight, based on the total weight of the oral formulation. The disintegrant may be used in an amount ranging from 1 to 30 % by weight, 1 to 20 % by weight, 1 to 100 % by weight or 1 to 5 % by weight, based on the total weight of the oral formulation. The binder may be used in an amount ranging from 1 to 30 % by weight, 1 to 20 % by weight, 1 to 100 % by weight, or 1 to 5 % by weight, based on the total weight of the oral formulation. The stabilizer may be used in an amount ranging from 0.1 to 10 % by weight, 0.3 to 5 % by weight, or 0.5 to 4 % by weight, based on the total weight of the oral formulation. The lubricant may be used in an amount ranging from 0.1 to 10 % by weight, 0.3 to 5 % by weight, or 0.5 to 4 % by weight, based on the total weight of the oral formulation.

For example, the diluent may be selected from the group consisting of microcrystalline cellulose (MCC), lactose monohydrate, lactose anhydrous, lactose, starch, mannitol, carboxymethyl cellulose, sorbitol, and combinations thereof, but is not limited thereto. The disintegrant may be selected from the group consisting of low-substituted hydroxypropyl cellulose, crospovidone, croscarmellose sodium, sodium starch glycolate, F-melt®, and combinations thereof, but is not limited thereto. The binder may be selected from the group consisting of hydroxypropyl cellulose, hydroxypropyl methylcellulose, hypromellose, polyvinyl acetic acid, povidone, polyvinylpyrrolidone, copovidone, Macrogol, sodium lauryl sulfate, light anhydrous silicic acid, synthetic aluminum silicate, calcium silicate or silicate derivatives such as magnesium

metasilicate aluminate, phosphate such as calcium hydrogen phosphate, carbonate such as calcium carbonate, pregelatinized starch, gums such as acacia gum, gelatin, cellulose derivatives such as ethyl cellulose, and mixtures thereof, but is not limited thereto. The stabilizer may be selected from the group consisting of BHT, BHA, ascorbic acid, tocopherol, EDTA, and mixtures thereof, but is not limited thereto. The lubricant may be selected from the group consisting of magnesium stearate, silicon dioxide, talc, light anhydrous silicic acid, sodium stearyl fumarate, and combinations thereof, but is not limited thereto.

The oral formulation can be administered once a day and can be taken daily.

The content of API comprised in the oral formulation is 30 to 50 % by weight, 35 to 50 % by weight, 40 to 50 % by weight, 45 to 50 % by weight, 30 to 45 % by weight, 35 to 45 % by weight, 40 to 45 % by weight, 30 to 40 % by weight, or 35 to 40 % by weight based on the total weight of the oral formulation.

In addition, the API may be comprised in an amount of, for example, 50 to 500 mg, 50 to 400 mg, 50 to 300 mg, 50 to 200 mg, 50 to 100 mg, 100 to 500 mg, 100 to 400 mg, 100 to 300 mg, 100 to 200 mg, 200 to 500 mg, 200 to 400 mg, 200 to 300 mg, 300 to 500 mg, or 300 to 400 mg per unit dosage form.

The API may be comprised in an amount of, for example, 50 mg, 100 mg, 150 mg, 200 mg, 300 mg, 400 mg, or 455 mg per unit dosage form.

The API assay in the formulation of the present invention may be 95% to 105%, 96% to 105%, 97% to 105%, 98% to 105%, 99% to 105%, 100% to 105%, 95% to 100%, 96% to 100%, 97% to 100%, 97% to 100%, or 99% to 100%. In addition, the API assay may be 95% or more, 96% or more, 97% or more, 98% or more, or 99% or more, and may be 105% or less, 104% or less, 103% or less, 102% or less, 101% or less, or 100% or less.

The "API assay" refers to the content of pure API excluding impurities among API content comprised per unit dosage form. Specifically, it may have the similar meaning as the purity of the API.

The present invention provides a method for preparing an oral formulation comprising 1-(3-cyano-1-isopropyl-indol-5-yl)pyrazol-4-carboxylic acid or a pharmaceutically acceptable salt thereof, which comprises the steps of i) mixing an API selected from 1-(3-cyano-1-isopropyl-indol-5-yl)pyrazol-4-carboxylic acid or a pharmaceutically acceptable salt thereof, and a glidant to preparing a first mixture; ii) mixing pharmaceutically acceptable excipients to preparing a second mixture; and iii) formulating said first mixture and said second mixture into an oral formulation.

The steps of preparing each of the first mixture and the second mixture in the preparation method of the present invention includes a step of milling after mixing.

The glidant in the first mixture in the manufacturing method of the present invention includes $SiO_2$ selected from the group consisting of colloidal silicon dioxide, hydrated silicon dioxide and combinations thereof.

The pharmaceutically acceptable excipients in the second mixture in the preparation method of the present invention optionally include the aforementioned excipients.

The API assay and assay of the formulation prepared by the preparation method of the present invention are the same as the aforementioned API assay and assay of the oral formulation of the present invention, respectively.

The oral formulation may be formulated according to any tablet preparation method or granule preparation method known in the art.

In addition, the present invention provides a method for treating or preventing human xanthine oxidase-related diseases by administration of an oral formulation comprising the 1-(3-cyano-1-isopropyl-indol-5-yl)pyrazol-4-carboxylic acid or a pharmaceutically acceptable salt thereof as an API and a glidant.

As used in the present invention, the term "human xanthine oxidase-related diseases" refers to diseases that can be treated or prevented by inhibiting human xanthine oxidase, and may be, for example, hyperuricacidemia, gout, heart failure, cardiovascular diseases, high blood pressure, diabetes, diabetes-related complications, kidney diseases, inflammation, joint diseases, inflammatory bowel disease, and the like, but is not limited thereto. Examples of the diabetes-related complications may be hyperlipidemia, arteriosclerosis, obesity, high blood pressure, retinopathy, renal failure, and the like.

The term "treatment" means stopping or delaying the progression of a disease when used for a subject showing symptoms of the disease, and the term "prevention" means stopping or delaying the symptoms of the disease when administered to a subject who does not show symptoms of the disease but is at a high risk of developing the disease.

Unless otherwise indicated, it is to be understood that all numbers used in the specification and claims, whether recited or not, may be modified in all instances by the term "about." It is also to be understood that the precise numbers used in the specification and claims form further embodiments of the present disclosure. Efforts have been made to ensure the accuracy of the numerical values disclosed in Examples. However, all measured values may inherently contain certain error values generated from the standard deviations measured by their respective measurement techniques.

**[Example and Experimental Example]**

Various evaluations in Examples and Comparative Examples were performed as follows.

**[Flowability analysis]**

The bulk density and tapped density of the mixture (powder) for preparing the uncoated tablets of Examples and Comparative Examples were measured. The bulk density was the volume when about 50 g of granular powder was put into the measuring cylinder, and the tapped density was measured when there was no further change in volume after lightly tapping the measuring cylinder on the floor at a constant height 100 times. The bulk density refers to the volume occupied by a certain mass of powder, and means the sum of the volume of powder and the volume of voids between particles as a total volume.
The flowability of the mixture was calculated as Carr's index in Equation (1) using the bulk density and tapped density measured above according to the method of Jinapong et al (2008).

$$\text{Equation (1)}: \quad CI = \frac{\rho_{tapped} - \rho_{bulk}}{\rho_{tapped}} \times 100$$

Carr's index is a measure of the compressibility of a powder and is defined as a percentage of (tapped density-bulk density)/tapped density. A higher index means that the powder is more compact and has poor flowability.
The table below shows the classification of powder flowability according to Carr's index.

**Table 1:**

| Carr's index (flowability) % | Flowability |
|---|---|
| <10 | Excellent |
| 11-15 | Good |
| 16-20 | Fair |
| 21-25 | Passable |
| 26-31 | Poor |
| 32-38 | Very poor |
| >38 | Very, very poor |

**[Hardness and friability analysis]**

The hardness and friability are used as indices that can predict the wear and tear of tablets in the handling and coating process of uncoated tablets after tableting.
For the friability test, tablets in an amount close to about 5 g are taken, the powder adhering to the tablets is removed, the mass is precisely measured, and then the tablets are placed in the drum of the friability tester. After rotating the drum 100 times, the tablets are taken out, and the powder adhering to the tablets is removed as in the beginning, and the mass is precisely measured.

$$\text{Friability (\%)} = (\text{mass before test} - \text{mass after test}) / (\text{mass before test}) \times 100$$

In the case of hardness test, one tablet is put into a table hardness tester to measure hardness, and in this way, 10 tablets are repeated to measure the average hardness.

**[Disintegration time analysis]**

The disintegration time is an index that affects dissolution and absorption in the body after ingestion of the formulation.
After the disintegration tester was sufficiently filled with purified water, it is adjusted to 37±2°C. Four tablets of the sample are put into the square glass pipe of the disintegration tester, it is operated in the prescribed way, and the time at which the tablet completely disintegrates and disappears is measured to obtain an average value.

**[Assay analysis]**

An assay test was conducted to evaluate the assay of the formulated formulation. The assay is an important quality characteristic of the final product, and the API average assay of the uncoated tablet was measured.

**<Analysis condition>**

Preparation of mobile phase: acetonitrile (500 mℓ) + purified water (500 mℓ) + TFA (1 mℓ)

Preparation of diluent: methanol (900 mℓ) + purified water (100 mℓ)

Preparation of standard solutions and test solutions: After completely dissolving the standards and samples in the diluent, they are analyzed according to the UPLC analysis method below.

Column: Waters CSH C18(2.1 mm I.D. X 100 mm L, Particle size 1.7 μm

Column temperature: 40°C

Mobile phase: acetonitrile/H2O/TFA = 500/500/1(v/v/v)

Flow rate: 0.35 mℓ/min

Detection: 258nm uv

Sample amount: 1 μℓ

Analysis time: 6 min

**[Dissolution rate analysis]**

According to the dissolution test method of the 10th revision of the Korean Pharmacopoeia, the uncoated tablets of Examples and Comparative Examples below were tested for dissolution. The dissolution method was a paddle method, the stirring speed was 50 rpm, and the dissolution temperature was 37±0.5°C. The eluate was 900 ml of pH 6.8 phosphate buffer.

For the analysis conditions, the solution obtained in the above dissolution test was filtered through a 0.45 μm membrane filter and the UPLC method was used, and the concentration of 1-(3-cyano-1-isopropyl-indol-5-yl)pyrazol-4-carboxylic acid, which is an API, was analyzed.

**<Analysis condition>**

The analysis conditions are the same as the assay analysis method.

**[Examples 1 to 8 and Comparative Example 1]**

The formulations of Examples 1 to 8 and Comparative Example 1 were prepared as uncoated tablets using the ingredients shown in Tables 2 to 4 below in the corresponding content.

Specifically, 1-(3-cyano-1-isopropyl-indol-5-yl)pyrazol-4-carboxylic acid (API) and glidant are mixed and milled

to prepare a first mixture. A diluent, a disintegrant, and a lubricant, which are components not included in the first mixture, are mixed and milled to prepare a second mixture.

In the case of Examples 1 to 8 comprising the glidant, after mixing the first mixture and the second mixture, the mixture is tableted using a rotary tablet machine (Modul P, GEA, Belgium) under the conditions of pre-pressure: 5.0 kN and main pressure: 14-15 kN to obtain uncoated tablets. In the case of Comparative Example 1 that does not comprise the glidant, the process of preparing mixture 1 is omitted, and 1-(3-cyano-1-isopropyl-indol-5-yl)pyrazol-4-carboxylic acid (API) and all excipients were mixed and milled, followed by tableting in the same way as Example 1, to prepare uncoated tablets.

(If colloidal silicon dioxide is used as a glidant, it is indicated as "$SiO_2$." MCC 102 used as a diluent is a microcrystalline cellulose component. PRUV® used as a lubricant is a trade name and is a sodium stearyl fumarate component) .

**Table 2:**

| Item | Ingredient | Example 1 | | Example 2 | | Example 3 | | Example 4 | |
|---|---|---|---|---|---|---|---|---|---|
| | | Content mg/T | Content ratio % | Content mg/T | Content ratio % | Content mg/T | Content ratio % | Content mg/T | Content ratio % |
| API | | 100. 0 | 45.5 | 100. 0 | 45.5 | 100. 0 | 45.5 | 100. 0 | 45.5 |
| Diluent | MCC 102 (microcrys talline cellulose) | 105. 4 | 47.9 | 104. 8 | 47.6 | 104. 1 | 47.3 | 103. 7 | 47.1 |
| Disintegrant | Crospovido ne | 9.7 | 4.4 | 9.7 | 4.4 | 9.7 | 4.4 | 9.7 | 4.4 |
| Glidant | Colloidal silicon dioxide ($SiO_2$) | 0.5 | 0.2 | 1.1 | 0.5 | 1.8 | 0.8 | 2.2 | 1.0 |
| Lubricant | PRUV® | 4.4 | 2.0 | 4.4 | 2.0 | 4.4 | 2.0 | 4.4 | 2.0 |
| Total weight of tablets (mg) | | 220. 0 | 100. 0 | 220. 0 | 100. 0 | 220. 0 | 100. 0 | 220. 0 | 100. 0 |

**Table 3:**

| Function | Type | Example 5 | | Example 6 | | Example 7 | | Example 8 | |
|---|---|---|---|---|---|---|---|---|---|
| | | Content mg/T | Content ratio % | Content mg/T | Content ratio % | Content mg/T | Content ratio % | Content mg/T | Content ratio % |
| API | | 100. 0 | 45.5 | 100. 0 | 50.0 | 100. 0 | 54.9 | 100. 0 | 59.9 |
| Diluent | MCC 102 (microcrys talline cellulose) | 104. 8 | 47.6 | 84.8 | 42.4 | 66.8 | 36.7 | 51.8 | 31.0 |
| Disintegrant | Crospovido ne | 9.7 | 4.4 | 9.7 | 4.9 | 9.7 | 5.3 | 9.7 | 5.8 |
| Glid | Colloidal | - | - | 1.1 | 0.6 | 1.1 | 0.6 | 1.1 | 0.7 |
| ant | silicon dioxide($SiO_2$) | | | | | | | | |
| | Talc | 1.1 | 0.5 | - | - | - | - | - | - |
| Lubricant | PRUV® | 4.4 | 2.0 | 4.4 | 2.2 | 4.4 | 2.4 | 4.4 | 2.6 |

(continued)

| Function | Type | Example 5 | | Example 6 | | Example 7 | | Example 8 | |
|---|---|---|---|---|---|---|---|---|---|
| | | Content mg/T | Content ratio % | Content mg/T | Content ratio % | Content mg/T | Content ratio % | Content mg/T | Content ratio % |
| Total weight of tablets (mg) | | 220. 0 | 100. 0 | 200. 0 | 100. 0 | 182. 0 | 100. 0 | 167. 0 | 100. 0 |

**Table 4:**

| Function | Type | Comparative Example 1 | |
|---|---|---|---|
| | | Content mg/T | Content ratio % |
| API | | 100.0 | 45.5 |
| Diluent | MCC 102 (microcrystalline cellulose) | 105.9 | 48.1 |
| Disintegrant | Crospovidone | 9.7 | 4.4 |
| Lubricant | PRUV® | 4.4 | 2.0 |
| Total weight of tablets (mg) | | 220.0 | 100.0 |

**Experimental Example 1: Characteristic analysis of formulation depending on glidant content**

The physical properties of the oral formulation comprising 1-(3-cyano-1-isopropyl-indol-5-yl)pyrazol-4-carboxylic acid as an API, depending on the inclusion and content of a glidant of $SiO_2$ as an excipient were analyzed by the aforementioned analysis method.

Specifically, the physical properties of the mixture state before formulation (bulk density, tapped density and Carr's index) and the physical properties of the uncoated tablet state after tableting the mixture (hardness, brittleness, disintegration time, assay and dissolution rate) were analyzed. The results are summarized in Table 5 below.

The bulk density, tapped density, and Carr's index did not show any significant difference depending on the inclusion and content of the glidant ($SiO_2$) in the mixture state before the uncoated tablet was prepared.

It has been known that when glidant is added to the conventional pharmaceutical formulation, the flowability is improved. However, in the case of the API (1-(3-cyano-1-isopropyl-indol-5-yl)pyrazol-4-carboxylic acid) of the present invention, the flowability was not significantly increased even when the glidant was comprised.

However, as a result of measuring the hardness and friability of the uncoated tablet, when $SiO_2$ is comprised (Examples 1 to 4) compared to the case without $SiO_2$ (Comparative Example 1), the hardness is 6.7 kP or higher, which satisfies 6.0 kP which is a target hardness of the uncoated tablet. On the other hand, in the case of Comparative Example 1, it was only 5.4 kP, which did not reach the target hardness of the uncoated tablet, making it unsuitable for formulation into tablets (Table 5).

In addition, as a result of measuring the API assay of the uncoated tablet, as shown in Table 6 and Figure 1 below, in the case of Comparative Example 1, the average assay is 95.3%, and when comprising 0.2 to 0.8% by weight of $SiO_2$, it is increased up to 98.8 to 99.4% (Examples 1 to 3). However, in the case of Example 4 comprising 1% by weight of $SiO_2$, it is reduced to 94.8% similar to Comparative Example 1, indicating that the preferred content range of $SiO_2$ is 0.2 to 0.8% by weight based on the total weight of the formulation.

Next, in the case of dissolution rate, Comparative Example 1 has a low hardness, so it shows early dissolution, but shows a constant dissolution rate thereafter. However, when $SiO_2$ was comprised, the slope showed a constant increase, and in particular, Example 2 (0.5 % by weight of $SiO_2$) showed the highest tendency for the 60-minute dissolution rate (Table 5).

**Table 5**

| Item | Example 1 | Example 2 | Example 3 | Example 4 | Comparative Example 1 |
|---|---|---|---|---|---|
| $SiO_2$ content (%) (based on total tablet) | 0.2 | 0.5 | 0.8 | 1 | 0 |
| Bulk density (g/ml) | 0.44 | 0.39 | 0.41 | 0.41 | 0.4 |

(continued)

| Item | | Example 1 | Example 2 | Example 3 | Example 4 | Comparative Example 1 |
|---|---|---|---|---|---|---|
| Carr's index (%) | | 23.0 | 25.0 | 24.0 | 24.0 | 25.0 |
| Hardness (kP) | | 6.7 | 7.3 | 7.5 | 7.2 | 5.4 |
| Friability (%) | | 0.15 | 0.05 | 0.04 | 0.03 | 0.05 |
| Disintegration time (sec) | | 12 | 10 | 10 | 12 | 10 |
| Assay | Average assay (%) | 98.8 | 99.4 | 99.1 | 94.8 | 95.3 |
| Dissolution rate | Average dissolution rate (15 min) (%) | 68.3 | 71.8 | 66.1 | 67.9 | 84.0 |
| | Average dissolution rate (30 min) (%) | 82.9 | 88.6 | 82.2 | 80.9 | 88.5 |
| | Average dissolution rate (60 min) (%) | 86.9 | 95.8 | 87.8 | 85.9 | 88.8 |

**Experimental Example 2: Characteristic analysis of formulation depending on type of glidant**

In order to analyze the characteristics depending on the types of glidant, the physical properties of example 2 and example 5 were analyzed. Example 2 shows best physical properties in table 5, and Example 5 was prepared by changing $SiO_2$ to talc while maintaining the glidant content (0.5 % by weight) from Example 2.

As shown in Table 6 below, in the case of Example 5 comprising talc as a glidant in the mixture state before the uncoated tablet was prepared, the bulk density showed no difference compared with Example 2 and Comparative Example 1, but Carr's index was calculated to be 28%. Therefore, in the case of Example 5, Carr's index is "poor" category and is not suitable for direct pressing. In addition, in the case of Example 5, the hardness of the uncoated tablet is 5.1 kP, which does not reach the target hardness of 6.0 kP, and the friability is also three times higher than in Example 2, and thus it has physical properties that are not suitable for the tablet.

It can be seen that the average assay of Example 5 is also lowered compared to Example 2.

Therefore, it can be seen that when $SiO_2$ is used as a glidant, the overall physical properties are improved compared to talc.

**Table 6:**

| Item | | Example 2 | Example 5 |
|---|---|---|---|
| Glidant content (%) (based on total tablet) | | 0.5 | 0.5 |
| Bulk density (g/ml) | | 0.39 | 0.41 |
| Carr's index (%) | | 25.0 | 28.0 |
| Hardness (kP) | | 7.3 | 5.1 |
| Friability (%) | | 0.05 | 0.14 |
| Disintegration time (sec) | | 10 | 8 |
| Assay | Average assay (%) | 99.4 | 96.5 |

**Experimental Example 3: Characteristic analysis depending on API content**

In order to prepare an uncoated tablet comprising an API of 50 % by weight or more, the characteristics depending on the increase of API content (Examples 6 to 8) based on the content (45.5 % by weight) of Example 2 were analyzed.

According to Table 7, since the density of the API is increased compared to the diluent as the API content is increased, the bulk density tends to slightly increase as the API content is increased, but the flowability itself does not show a significant difference.

However, as the API content is increased, the hardness of the uncoated tablet is weakened and friability is

increased due to the decrease in tableting properties (reduction in compressibility of the tablet), and in particular, Example 8 (API content: 60 % by weight) showed a hardness of 3.0 kP and a friability of 0.2% or more, and thus it was not suitable for formulating into tablets (Table 7) .

Also, as a result of measuring the API assay of the uncoated tablet, Examples 2, 6, and 7 showed an average assay of 99% or more, whereas Example 8 showed an average assay of 94.6% (Table 7).

Taken together, when the API content is 55% by weight or less, the average assay is 99% or more, but when the API content is 60% by weight, the average assay is significantly lowered to 94.6%, and when the API content is more than 50%, even if the content is excellent, the hardness of the uncoated tablet is lowered and the friability is increased. Therefore, it can be seen that when the content of the API is 55% by weight or more, it is not desirable to formulate it as a tablet.

**Table 7:**

| Item | | Example 2 | Example 6 | Example 7 | Example 8 |
|---|---|---|---|---|---|
| API content (%) (based on total tablet) | | 45.5 | 50 | 55 | 60 |
| Bulk density (g/ml) | | 0.39 | 0.43 | 0.43 | 0.44 |
| Carr's index (%) | | 25.0 | 25.0 | 24.0 | 23.0 |
| Hardness (kP) | | 7.3 | 5.4 | 4.0 | 3.0 |
| Friability (%) | | 0.05 | 0.08 | 0.10 | 0.22 |
| Disintegration time (sec) | | 10 | 10 | 10 | 7 |
| Assay | Average assay (%) | 99.4 | 99.6 | 99.3 | 94.6 |

Up to now, preferred examples of the present invention have been mainly looked at. It will be understood by those of ordinary skill in the art to which the present invention pertains that the present invention can be implemented in a modified form without departing from the essential characteristics of the present invention. Therefore, the examples disclosed above are to be considered in an illustrative rather than a restrictive sense. The scope of the present invention is indicated in the claims rather than the foregoing description, and all differences within the scope equivalent thereto should be construed as being included in the present invention.

## Claims

1. An oral formulation comprising an active pharmaceutical ingredient (API) selected from 1-(3-cyano-1-isopropyl-indol-5-yl)pyrazol-4-carboxylic acid or a pharmaceutically acceptable salt thereof; and a glidant.

2. The oral formulation according to claim 1, wherein the glidant is selected from the group consisting of colloidal silicon dioxide, hydrated silicon dioxide and combinations thereof.

3. The oral formulation according to claim 2, wherein the content of the glidant is greater than 0% by weight and less than 1% by weight based on the total weight of the formulation.

4. The oral formulation according to claim 3, wherein the content of the glidant is 0.2 to 0.8% by weight based on the total weight of the formulation.

5. The oral formulation according to claim 1, wherein the oral formulation further comprises one or more excipients selected from a diluent, a disintegrant, a binder and a lubricant.

6. The oral formulation according to claim 1, wherein the content of the API is 30 to 55% by weight based on the total weight of the formulation.

7. The oral formulation according to claim 6, wherein the content of the API is 40 to 50% by weight based on the total weight of the formulation.

8. The oral formulation according to claim 1, wherein the content of the API is 50 mg, 100 mg, 200 mg, or 300 mg per unit dosage form.

**9.** The oral formulation according to any one of claims 1 to 8, wherein the assay of the API is 97% or more.

**10.** A method for preparing an oral formulation comprising 1-(3-cyano-1-isopropyl-indol-5-yl)pyrazol-4-carboxylic acid or a pharmaceutically acceptable salt thereof, which comprises the steps of

i) mixing 1-(3-cyano-1-isopropyl-indol-5-yl)pyrazol-4-carboxylic acid or a pharmaceutically acceptable salt thereof as an API; and a glidant to preparing a first mixture;
ii) mixing pharmaceutically acceptable excipients to preparing a second mixture; and
iii) formulating said first mixture and said second mixture into an oral formulation.

**11.** The method for preparing an oral formulation according to claim 10, wherein the content of the glidant is 0.2 to 0.8% by weight based on the total weight of the formulation.

**12.** The method for preparing an oral formulation according to claim 10, wherein the content of the API is 40 to 50% by weight based on the total weight of the formulation.

**13.** An oral formulation prepared by the method of any one of claims 10 to 12, wherein the assay of the above API is 97% or more.

【Figure 1】

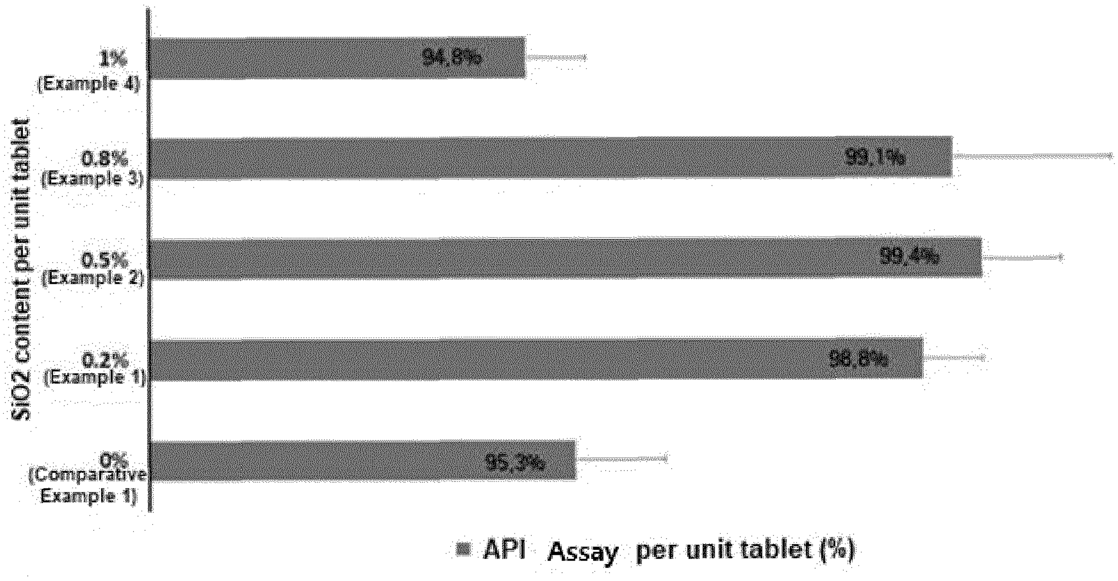
SiO2 content per unit tablet
1%
(Example 4)
0.8%
(Example 3)
0.5%
(Example 2)
0.2%
(Example 1)
0%
(Comparative
Example 1)
94.8%
99.1%
99.4%
98.8%
95.3%
API Assay per unit tablet (%)

**INTERNATIONAL SEARCH REPORT**

International application No.
**PCT/KR2021/017843**

**A. CLASSIFICATION OF SUBJECT MATTER**

**A61K 9/20**(2006.01)i; **A61K 31/4155**(2006.01)i

According to International Patent Classification (IPC) or to both national classification and IPC

**B. FIELDS SEARCHED**

Minimum documentation searched (classification system followed by classification symbols)

A61K 9/20(2006.01); A61K 31/4155(2006.01); A61K 9/28(2006.01); C07D 403/04(2006.01); C07D 417/04(2006.01); C07D 491/056(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 1-(3-시아노-1-아이소프로필-인돌-5-일)피라졸-4-카르복실산, 경구 (oral), 유동화제 (glidant), 약학조성물 (pharmaceutical composition)

**C. DOCUMENTS CONSIDERED TO BE RELEVANT**

| Category* | Citation of document, with indication, where appropriate, of the relevant passages | Relevant to claim No. |
|---|---|---|
| DY | KR 10-2012-0114174 A (LG LIFE SCIENCES LTD.) 16 October 2012 (2012-10-16)<br>See abstract; and paragraphs [0001]-[0003], [0006], [0025] and [0029]. | 1-13 |
| Y | US 5879706 A (CARTER, B. H. et al.) 09 March 1999 (1999-03-09)<br>See abstract; and claims 1, 6 and 15. | 1-13 |
| DY | KR 10-2011-0037883 A (LG LIFE SCIENCES LTD.) 13 April 2011 (2011-04-13)<br>See abstract; and paragraphs [0187]-[0198] and [0204]. | 1-13 |
| A | MAJEROVA, D. et al. Effect of colloidal silica on rheological properties of common pharmaceutical excipients. European Journal of Pharmaceutics and Biopharmaceutics. 2016, vol. 106, pp. 2-8.<br>See entire document. | 1-13 |
| A | KR 10-2009-0128488 A (KISSEI PHARMACEUTICAL CO., LTD.) 15 December 2009 (2009-12-15)<br>See entire document. | 1-13 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 March 2022** | **10 March 2022** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2019)

**INTERNATIONAL SEARCH REPORT**
**Information on patent family members**

International application No.
**PCT/KR2021/017843**

| Patent document cited in search report | Publication date (day/month/year) | Patent family member(s) | Publication date (day/month/year) |
|---|---|---|---|
| KR 10-2012-0114174 A | 16 October 2012 | CN 103459381 A | 18 December 2013 |
| | | CN 103459381 B | 09 December 2015 |
| | | JP 2014-510133 A | 24 April 2014 |
| | | JP 6008937 B2 | 19 October 2016 |
| | | KR 10-1424013 B1 | 18 August 2014 |
| | | TW 201245182 A | 16 November 2012 |
| | | TW I548630 B | 11 September 2016 |
| | | WO 2012-138147 A2 | 11 October 2012 |
| | | WO 2012-138147 A3 | 29 November 2012 |
| US 5879706 A | 09 March 1999 | AP 666 A | 24 August 1998 |
| | | AT 190483 T | 15 April 2000 |
| | | BG 101831 A | 30 April 1998 |
| | | BG 63187 B1 | 29 June 2001 |
| | | BR 9606769 A | 30 December 1997 |
| | | CA 2210891 A1 | 25 July 1996 |
| | | CA 2210891 C | 19 May 2009 |
| | | CN 100131026 C | 17 December 2003 |
| | | CN 100179100 A | 15 April 1998 |
| | | CN 100313081 C | 02 May 2007 |
| | | CN 100494898 A | 12 May 2004 |
| | | CY 2182 B1 | 23 August 2002 |
| | | CZ 229297 A3 | 18 February 1998 |
| | | CZ 296514 B6 | 15 March 2006 |
| | | DE 69607146 T2 | 21 September 2000 |
| | | DK 0806943 T3 | 24 July 2000 |
| | | EP 0806943 A1 | 19 November 1997 |
| | | EP 0806943 B1 | 15 March 2000 |
| | | ES 2145425 T3 | 01 July 2000 |
| | | GE P20022752 B | 10 May 2002 |
| | | GR 3033677 T3 | 31 October 2000 |
| | | HK 1002851 A1 | 25 September 1998 |
| | | IL 116830 A | 29 February 2000 |
| | | IN 181318 B | 09 May 1998 |
| | | JP 10-512564 A | 02 December 1998 |
| | | JP 3350055 B2 | 25 November 2002 |
| | | KR 10-0412298 B1 | 21 April 2004 |
| | | KR 10-1998-0701524 A | 15 May 1998 |
| | | NO 973327 B1 | 03 December 2001 |
| | | NO 973327 L | 16 September 1997 |
| | | NZ 298846 A | 26 June 1998 |
| | | OA 10500 A | 10 April 2002 |
| | | PL 185307 B1 | 30 April 2003 |
| | | PL 321361 A1 | 08 December 1997 |
| | | PT 806943 E | 31 August 2000 |
| | | RO 118175 B1 | 28 March 2003 |
| | | SK 282030 B6 | 08 October 2001 |
| | | SK 96497 A3 | 14 January 1998 |
| | | TR 199700657 T1 | 21 February 1998 |
| | | UA 68325 C2 | 16 August 2004 |
| | | UY 25774 A1 | 31 October 2000 |

Form PCT/ISA/210 (patent family annex) (July 2019)

**INTERNATIONAL SEARCH REPORT**
**Information on patent family members**

International application No.
**PCT/KR2021/017843**

| Patent document cited in search report | Publication date (day/month/year) | Patent family member(s) | Publication date (day/month/year) |
|---|---|---|---|
| | | UY 25778 A1 | 21 August 2000 |
| | | WO 96-22082 A1 | 25 July 1996 |
| | | ZA 96448 B | 07 August 1996 |
| KR 10-2011-0037883 A | 13 April 2011 | AP 3346 A | 31 July 2015 |
| | | AR 078504 A1 | 09 November 2011 |
| | | AU 2010-304091 A1 | 19 April 2012 |
| | | AU 2010-304091 B2 | 09 June 2016 |
| | | BR 112012007828 A2 | 08 March 2016 |
| | | BR 112012007828 B1 | 12 January 2021 |
| | | BR 112012007828 B8 | 25 May 2021 |
| | | CA 2774133 A1 | 14 April 2011 |
| | | CA 2774133 C | 25 June 2019 |
| | | CL 2012000738 A1 | 14 September 2012 |
| | | CN 102574839 A | 11 July 2012 |
| | | CN 102574839 B | 25 November 2015 |
| | | CO 6430501 A2 | 30 April 2012 |
| | | EA 021025 B1 | 31 March 2015 |
| | | EA 201270520 A1 | 30 October 2012 |
| | | EC SP12011793 A | 30 May 2012 |
| | | EP 2467378 A2 | 27 June 2012 |
| | | EP 2467378 B1 | 27 July 2016 |
| | | ES 2599829 T3 | 03 February 2017 |
| | | HK 1170224 A1 | 22 February 2013 |
| | | IL 218669 A | 31 May 2012 |
| | | JP 2013-507355 A | 04 March 2013 |
| | | JP 5702392 B2 | 15 April 2015 |
| | | KR 10-1751325 B1 | 27 June 2017 |
| | | MA 33880 B1 | 02 January 2013 |
| | | MX 2012003782 A | 31 August 2012 |
| | | MY 162813 A | 31 July 2017 |
| | | PE 20121088 A1 | 17 August 2012 |
| | | SG 179186 A1 | 30 May 2012 |
| | | TR 201203989 T1 | 21 September 2012 |
| | | TW 201118077 A | 01 June 2011 |
| | | TW I423962 B | 21 January 2014 |
| | | UA 110197 C2 | 10 December 2015 |
| | | US 2012-0184582 A1 | 19 July 2012 |
| | | US 8729273 B2 | 20 May 2014 |
| | | WO 2011-043568 A2 | 14 April 2011 |
| | | WO 2011-043568 A3 | 29 September 2011 |
| | | ZA 201202544 B | 29 December 2012 |
| KR 10-2009-0128488 A | 15 December 2009 | AU 2008-239017 A1 | 23 October 2008 |
| | | AU 2008-239017 B2 | 27 September 2012 |
| | | BR PI0810524 A2 | 21 October 2014 |
| | | BR PI0810524 B1 | 10 September 2019 |
| | | BR PI0810524 B8 | 25 May 2021 |
| | | CA 2682391 A1 | 23 October 2008 |
| | | CA 2682391 C | 08 July 2014 |
| | | CN 101679251 A | 24 March 2010 |
| | | CN 101679251 B | 02 October 2013 |

Form PCT/ISA/210 (patent family annex) (July 2019)

**INTERNATIONAL SEARCH REPORT**
**Information on patent family members**

International application No.
**PCT/KR2021/017843**

| Patent document cited in search report | Publication date (day/month/year) | Patent family member(s) | Publication date (day/month/year) |
|---|---|---|---|
| | | CY 1114647 T1 | 14 December 2016 |
| | | DK 2133332 T3 | 30 September 2013 |
| | | EP 2133332 A1 | 16 December 2009 |
| | | EP 2133332 B1 | 18 September 2013 |
| | | EP 2402314 A1 | 04 January 2012 |
| | | EP 2402314 B1 | 31 July 2013 |
| | | ES 2431815 T3 | 28 November 2013 |
| | | HK 1140197 A1 | 08 October 2010 |
| | | HR P20130900 T1 | 08 November 2013 |
| | | JP 5330989 B2 | 30 October 2013 |
| | | KR 10-1502957 B1 | 16 March 2015 |
| | | MX 2009010966 A | 02 November 2009 |
| | | MX 337527 B | 09 March 2016 |
| | | PL 2133332 T3 | 28 February 2014 |
| | | PT 2133332 E | 03 October 2013 |
| | | RU 2009141614 A | 20 May 2011 |
| | | RU 2477274 C2 | 10 March 2013 |
| | | SI 2133332 T1 | 28 February 2014 |
| | | US 2010-0056521 A1 | 04 March 2010 |
| | | US 2011-0230454 A1 | 22 September 2011 |
| | | US 2013-0252955 A1 | 26 September 2013 |
| | | US 2014-0179932 A1 | 26 June 2014 |
| | | US 8003647 B2 | 23 August 2011 |
| | | US 8466152 B2 | 18 June 2013 |
| | | US 8829040 B2 | 09 September 2014 |
| | | US 8993616 B2 | 31 March 2015 |
| | | WO 2008-126898 A1 | 23 October 2008 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020200165790
- KR 101751325
- KR 101424013